# EUROPEAN PATENT APPLICATION

(11) **EP 1 607 042 A1**
(43) Date of publication of application: **21.12.2005**
(21) Application number: 04722957.0
(22) Date of filing: 24.03.2004
(51) Int. Cl.: A61B 5/145, A61B 5/15

(54) **HUMOR SAMPLING IMPLEMENT AND METHOD OF HUMOR SAMPLING**

(30) Priority: 27.03.2003 JP 2003088246
(71) Applicant: TERUMO KABUSHIKI KAISHA, Tokyo 151-0072 (JP)
(72) Inventor: YAZAKI, Hirofumi, Terumo Kabushiki Kaisha, Nakakoma-gun, Yamanashi 409-3853 (JP); NAKAMURA, Toshihisa, Terumo Kabushiki Kaisha, Nakakoma-gun, Yamanashi 409-3853 (JP); YAGUCHI, Yoshiaki, Terumo Kabushiki Kaisha, Nakakoma-gun, Yamanashi 409-3853 (JP)
(74) Representative: TBK-Patent
(86) International application number: PCT/JP2004/004094
(87) International publication number: WO 2004/084727

(57) **Abstract**

A humor sampling implement including a detection unit having a main frame part, the main frame part having a blood transfer channel provided to collect blood through a blood inflow port and transfer the blood to a blood outflow port, and a test paper for detecting glucose in the blood transferred through the blood transfer channel provided in the main frame part, wherein the main frame part is furnished with a convex part arranged so as to be in piled relationship with the test paper in plan view and protruding in the blood transfer channel toward the blood outflow port. In particular, the blood transfer channel includes a first blood transfer channel opening to the blood inflow port and a second blood transfer channel connected to the first blood transfer channel wherein the direction of blood transfer is different from that in the first blood transfer channel, and the convex part is provided at an end portion on blood outflow port side of the first blood transfer channel of the main frame part so as to protrude in the second blood transfer channel.

## Description

### Technical Field

The present invention relates to a humor sampling implement for use in the state of being mounted in a component measuring instrument used for measurement of blood sugar level, for example, and to a method of humor sampling for collecting humor at the time of, for example, examination or the like.

### Background Art

Hitherto, for measuring various components of blood, methods of measuring a reaction product of a specific enzyme capable of reacting with a specified component of blood have been investigated. Particularly, measurement of blood sugar level is important for monitoring the conditions of a patient, and self measurement of blood sugar consisting in monitoring the daily variation of blood sugar level by the patient himself has been recommended. Besides, in recent years, the number of diabetes patients has been increasing, and methods and means of measurement being simple and accompanied by little pain have been sought.

The measurement of blood sugar level is in many cases conducted by utilizing the oxidation of glucose (grape sugar) by an enzyme such as glucose oxidase and glucose dehydrogenase. At present, the measurement of blood sugar level is conducted by use of a blood sugar measuring instrument based on a colorimetric method in which a test paper capable of coloration according to the quantity of glucose in blood is mounted on the instrument, blood is supplied to the test paper and developed on the test paper so as to permit coloration, and the degree of coloration is optically measured (colorimetry) to thereby quantitatively detect the blood sugar level, an electrode type method in which the reaction product of the enzyme reaction is measured, or the like.

The colorimetric measurement of blood sugar level, in general, is conducted by use of a blood sugar measuring instrument to which a chip (humor sampling implement) with the test paper incorporated therein is mounted. The chip has a blood channel (humor transfer channel) through which blood (humor) collected via a blood inflow port is transferred to the test paper by utilizing capillarity.

Meanwhile, the blood has a comparatively high viscidity and may stagnate in the blood channel in some cases. Particularly, the stagnation of blood is liable to occur at a part near the test paper of the blood channel (on the opposite side of the blood inflow port), or, in the case of a blood channel in which the direction of blood transfer changes, the stagnation is liable to occur at the part where the blood transfer direction changes. In this case, it becomes impossible to measure the blood sugar level, the chip must be thrown away, and the patient is necessarily subjected to re-sampling of blood, which means an added burden on the patient.

In view of this, there has been proposed, for example in Japanese Patent Laid-open No. 2001-314394, a chip in which a convex part protruding in a blood channel toward the opposite side of the blood transfer direction is provided at the part where the direction of blood transfer in the blood channel changes. In this chip, with the convex part provided, it is intended to suppress the generation of meniscus in the vicinity of the convex part and to smoothly transfer the blood through capillarity.

In this chip, however, the convex part is provided on the blood inflow port side, and the countermeasure against the stagnation of blood at a part near the test paper is not satisfactorily contrived.

### Disclosure of Invention

There is a need for the prevent invention to provide a humor sampling implement which is capable of transferring humor to a detection unit more securely and speedily, and a method of humor sampling capable by which it is possible to sample humor more securely and speedily.

To attain the above need, according to the present invention, there is provided a humor sampling implement including:
a main frame part having a humor transfer channel provided to collect humor through a humor inflow port and transfer the humor to a humor outflow port; and
a detection part provided at the main frame part to detect a predetermined component of the humor transferred through the humor transfer channel; wherein
the main frame part is provided with a convex part arranged so as to be in overlapped relationship with the detection part in plan view and protruding in the humor transfer channel toward the humor outflow port.

By this it is possible to prevent the cross-sectional area of the humor transfer channel on the humor outflow port side from being increased, or to reduce the cross-sectional area; as a result, it is possible to prevent the efficiency of humor transfer from being lowered, or to enhance the efficiency of humor transfer. In addition, it is possible to favorably prevent or suppress the generation of meniscus in the vicinity of the convex part.

From the foregoing, it is possible to transfer the collected humor to the detection part more securely and speedily.

In the humor sampling implement according to the present invention, the convex part is preferably provided at a position corresponding substantially to a center of the detection part.

In the humor sampling implement according to the present invention, it is preferable that the humor transfer channel has a first humor transfer channel opening to the humor inflow port, and a second humor transfer channel connected to the first humor transfer channel, the second humor transfer channel being different from the first humor transfer channel in a direction humor transfer; and
the convex part is provided at an end portion on humor outflow port side of the first humor transfer channel of the main frame part so as to protrude in the second humor transfer channel.

In the humor sampling implement according to the present invention, it is preferable that the direction of humor transfer in the first humor transfer channel and the direction of humor transfer in the second humor transfer channel are substantially orthogonal to each other.

In the humor sampling implement according to the present invention, it is preferable that V₁/V₂ is in a range of from 0.04 to 0.7, where V₁ [mm³] is a volume of the convex part, and V₂ [mm³] is an inside volume of the second humor transfer channel.

In the humor sampling implement according to the present invention, the convex part preferably has a surface having been treated to be hydrophilic.

In the humor sampling implement according to the present invention, the humor transfer channel preferably has a cross-sectional area gradually decreasing part of which the cross-sectional area gradually decreases along the direction toward the humor outflow port.

In the humor sampling implement according to the present invention, the cross-sectional area gradually decreasing part preferably has an R₁/R₂ ratio in a range of from 0.3 to 0.8, where R₁ [mm²] is a minimum cross-sectional area, and R₂ [mm²] is a maximum cross-sectional area, of the cross-sectional area gradually decreasing part.

In the humor sampling implement according to the present invention, the cross-sectional area gradually decreasing part is preferably provided in the vicinity of the humor outflow port of the humor transfer channel.

In the humor sampling implement according to the present invention, the main frame part preferably has a lower member, and an upper member which is positioned on the lower member and which, together with the lower member, defines a part of the humor transfer channel.

The humor sampling implement according to the present invention preferably includes a puncture needle provided at its tip end with a sharp needle tip by which to puncture a skin, thereby causing the humor to flow out.

According to the present invention, there is provided a method of humor sampling in which a humor sampling implement as set forth in claim 1 is used.

According to the present invention, there is provided a method of humor sampling, including the step of collecting humor through a humor inflow port of a humor sampling implement, the humor sampling implement including:
a main frame part having a humor transfer channel provided to collect humor through the humor inflow port and transfer the humor to a humor outflow port; and
a detection part provided in the main frame part to detect a predetermined component of the humor transferred through the humor transfer channel; wherein
the main frame part is provided with a convex part arranged so as to be in overlapped relationship with the detection part in plan view and protruding in the humor transfer channel toward the humor outflow port.

### Brief Description of Drawings

Fig. 1 is a oblique perspective view of one embodiment of a chip (a humor sampling implement according to the present invention.
Fig. 2 is an exploded perspective view of the chip shown in Fig. 1.
Fig. 3 is a oblique perspective view, as viewed from the lower side, of the chip shown in Fig. 1.
Fig. 4 is a sectional view along line A-A of Fig. 1.
Fig. 5 is a sectional view along line B-B of Fig. 1.
Fig. 6 is a plan view of a detection unit possessed by the chip shown in Fig. 1.
Fig. 7 is a sectional view along line C-C of Fig. 6.
Fig. 8 is a plan view of a component measuring instrument used with the chip (the humor sampling implement according to the present invention) mounted therein.
Fig. 9 is a side view of the component measuring instrument shown in Fig. 8.
Fig. 10 is a sectional view along line X-X of Fig. 8.
Fig. 11 is a sectional view along line Y-Y of Fig. 9.
Fig. 12 is a sectional view along line Y-Y of Fig. 9.
Fig. 13 is a view of another configuration example of a second blood transfer channel (second humor transfer channel).

### Best Mode for Carrying out the Invention

Now, preferred embodiments of the humor sampling implement and the method of humor sampling according to the present invention will be described in detail below.

First, before describing the humor sampling implement and the method of humor sampling according to the present invention, a component measuring instrument used with the humor sampling implement of the invention mounted therein (thereto) will be described. In the following description, as the component measuring instrument, an instrument which includes a puncturing means and which is capable of measuring (detecting) a predetermined component of humor collected via a cuticle (skin) will be described as a representative.

In addition, the site concerning the sampling of the humor of the cuticle (humor sampling site) is preferably a finger, but it may, for example, be a hand (the palm, the back, a side part), an arm, a thigh, an earlobe or the like.

In the following description, blood as the humor, glucose (grape sugar) as the predetermined component, and a fingertip (finger) as the humor sampling site will be taken as representatives, respectively.

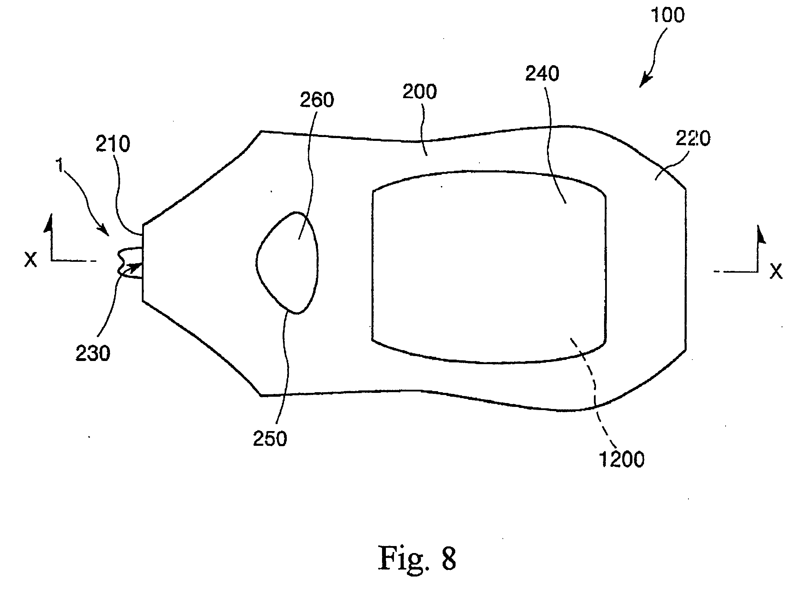

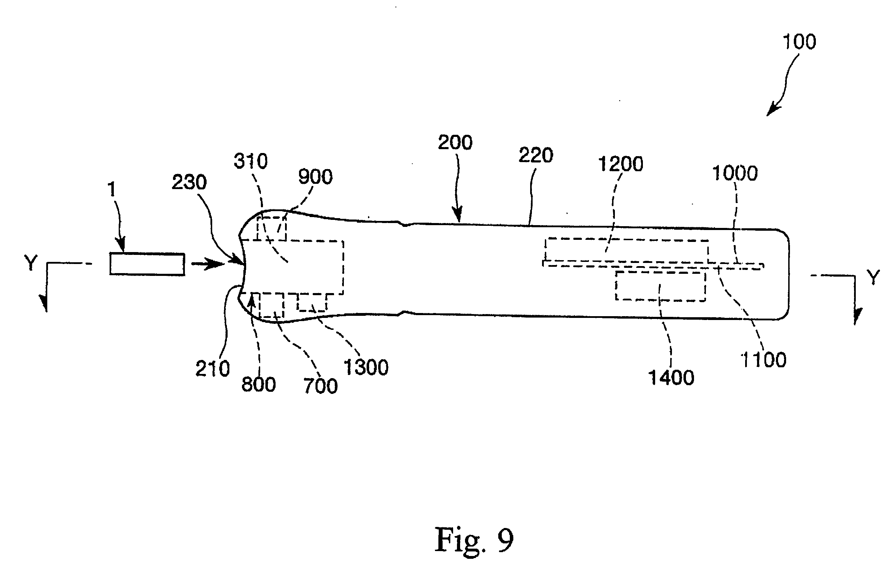

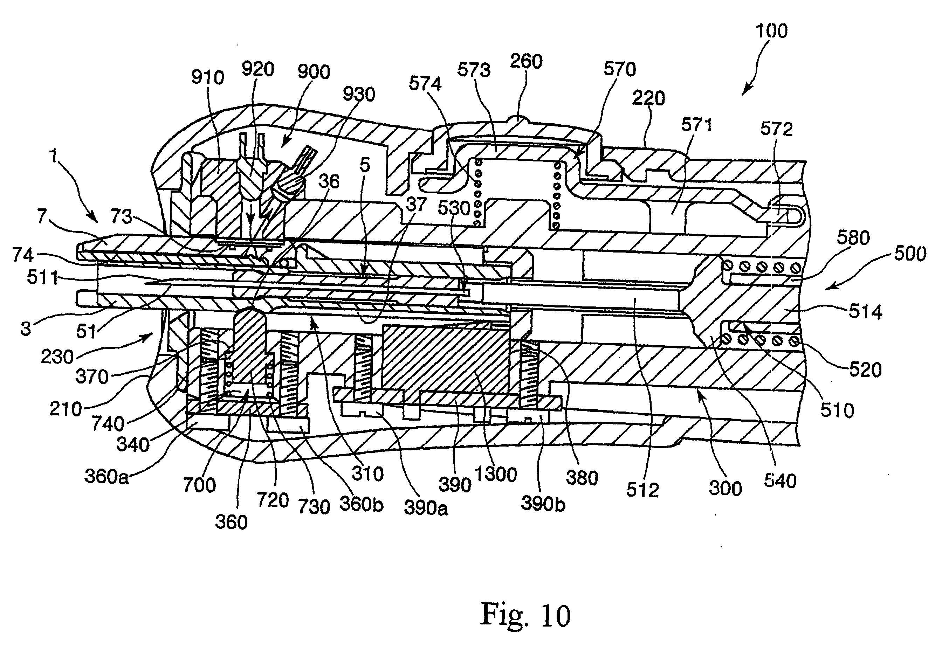

Fig. 8 is a plan view of the component measuring instrument used with a chip (the humor sampling implement according to the present invention) mounted therein, Fig. 9 is a side view of the component measuring instrument shown in Fig. 8, Fig. 10 is a sectional view along line X-X of Fig. 8, and Fig. 11 and Fig. 12 are each a sectional view along line Y-Y of Fig. 9. In the following description, in Figs. 8 to 12, the left side will be referred to as "the tip end", the right side as "the base end", and in Figs. 9 to 12, the upper side will be referred to as "the upper" or "the upper side", and the lower side as "the lower" or "the lower side".

The component measuring instrument (blood component measuring instrument) 100 shown in the figures is an instrument used with the chip (the humor sampling implement according to the present invention) 1 mounted thereto, and includes a main frame 200, a hold member 300 for containing and holding a puncturing means 500, a pushing mechanism 700, an ejecting mechanism 800, a measuring means 900, a control means 1100 provided on a circuit substrate 1000, a display unit 1200, a micro-switch 1300, and a battery (power supply unit) 1400. Now, the component elements will be described below.

The main frame 200 is box-like in shape, and contains therein the hold member 300, the pushing mechanism 700, the ejecting mechanism 800, the measuring means 900, the circuit substrate 1000 provided with the control means 1100, the display unit 1200, the micro-switch 1300 and the battery (power supply unit) 1400.

The main frame 200 is provided in its tip end face 210 with an opening 230 penetrating through the inside and outside of the main frame 200. The opening 230 is formed to correspond to the cross-sectional shape of the chip 1. The chip 1 is mounted in (mounted to) a chip mounting part 310 formed at a tip end portion of the hold member 300 via the opening 230. As a result, a condition where the chip 1 is mounted in the component measuring instrument 100 (hereinafter referred to as "the chip mounted condition") is obtained.

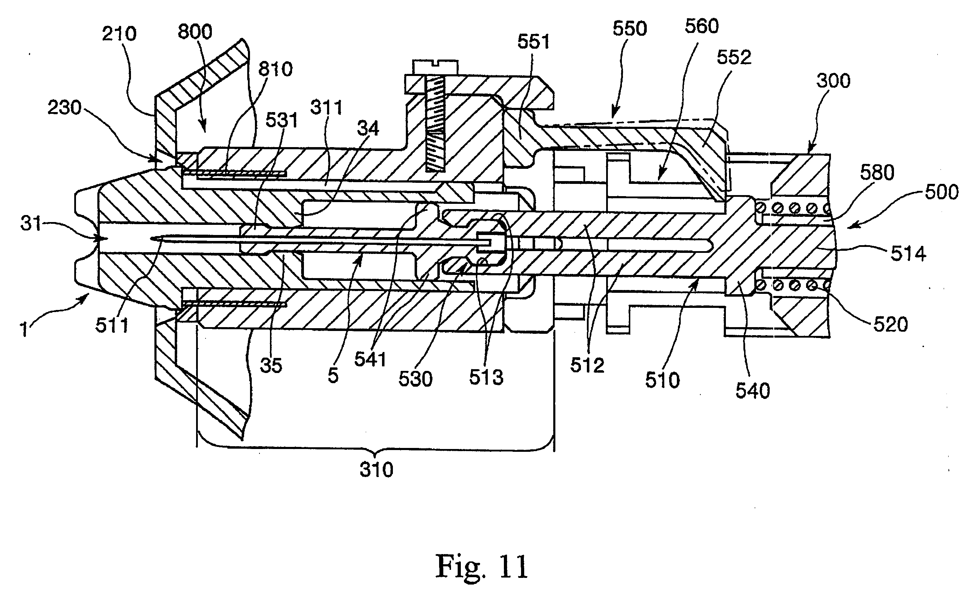

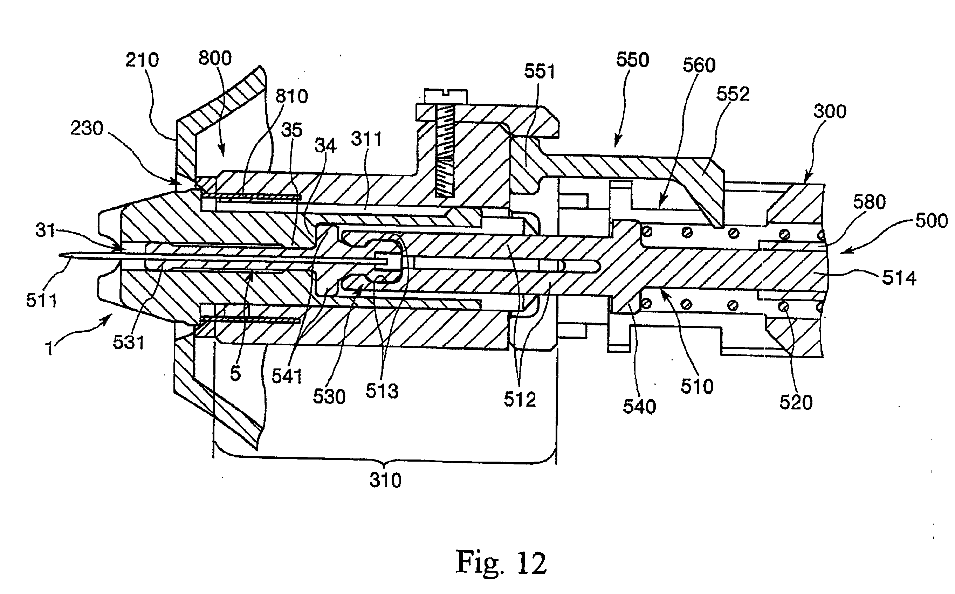

The chip mounting part 310 is provided in its right inside surface (in Figs. 11 and 12, the side surface on the upper side) with a groove 311 along the longitudinal direction thereof. At the time of mounting the chip 1 to the component measuring instrument 100, a rib 9 formed at a base end portion of a casing 3 which will be described later is inserted in the groove 311, and the groove 311 guides the rib 9.

In addition, the main frame 200 is moderately curved in shape on both sides thereof, whereby the component measuring instrument 100 can be gripped easily and securely.

The main frame 200 is provided in its upper surface 220 with a hole part 250, and an operation button 260 is provided in the hole part 250.

The component measuring instrument 100 is so configured that pushing of the operation button 260 causes the puncturing means 500 (described later) to operate. Incidentally, a configuration may be adopted in which pushing the operation button 260 turns ON the power supply for the component measuring instrument 100.

Besides, the upper surface 220 of the main frame 200 is provided on its base end side with a display window (opening) 240 penetrating through the inside and outside of the main frame 200, and the display window 240 is closed with a plate-like member formed of a transparent material.

The display unit 1200 is disposed on the lower side of the display window 240. Therefore, various kinds of information displayed on the display unit 1200 can be confirmed through the display window 240.

The display unit 1200 is composed, for example, of a liquid crystal display device (LCD) or the like. For example, ON/OFF of the power supply, the power supply voltage (residual power of the battery), the measured value, the measurement date and time, an error indication, an operation guidance and the like can be displayed on the display unit 1200.

In addition, the circuit substrate 1000 provided with the control means 1100 and the battery 1400 are provided on the lower side of the display unit 1200.

The control means 1100 is composed, for example, of a microcomputer, and controls the operations of the component measuring instrument 100, based on the decision of whether blood has been sampled or not, or the like. Besides, the control means 1100 incorporates an arithmetic unit for computing the glucose level (blood sugar level) in the blood based on a signal from the measuring means 900 which will be described later.

The battery 1400 is electrically connected to the measuring means 900, the control means 1100, the display unit 1200 and the micro-switch 1300 so as to supply electric power required for the operations of these components.

On the upper side of the hold member 300, the measuring means 900 is provided so as to face the chip mounting part 310. The measuring means 900 optically detects the supply (collection) of blood to a test paper (detection unit) 73 possessed by the chip 1, and optically measure the glucose level in the blood developed on the test paper 73. The measuring means 900 is composed of an optical block, and is disposed at a position facing the test paper 73 in the chip mounted condition (in the vicinity of a lateral side of the test paper 73).

Since the measuring means 900 thus has both the function of detecting the collection of the blood and the function of measuring the amount of glucose in the blood developed on the test paper 73, the number of component parts can be reduced and the configuration can be simplified, as compared with the case where the detecting means and the measuring means are provided separately. Besides, the number of assembling steps of the instrument can be reduced.

The control means 900 has a block body 910, and a light emitting device (light emitting diode) 920 and a light receiving device (photodiode) 930 which are fixed to the block body 910.

The light emitting device 920 is electrically connected to the control means 1100, and the light receiving device 930 is electrically connected to the control means 1100 through an amplifier and an A/D converter which are not shown in the figures.

The light emitting device 920 operates, to emit light, in response to a signal from the control means 1100. The light is preferably pulsed light which is intermittently emitted at a predetermined time interval.

When the light emitting device 920 is turned ON in the chip mounted condition, the light emitted from the light emitting device 920 is incident on the test paper 73, and the reflected light is received by the light receiving device 930, to be subjected to photo-electric conversion. An analog signal according to the amount of light received is outputted from the light receiving device 930, the signal is amplified in a desired manner by the amplifier, the amplified signal is then converted into a digital signal by the A/D converter, and the digital signal is inputted to the control means 1100.

Based on the signal inputted, the control means 1100 decides whether the blood has been sampled or not, i.e., whether the blood has been developed on the test paper 73 of the chip 1 or not.

Besides, based on the signal inputted, the control means 1100 performs a predetermined arithmetic processing, and performs correction calculation or the like as required, to determine the amount of glucose in the blood (blood sugar level). The blood sugar level thus determined is displayed on the display unit 1200.

The pushing mechanism 700 and the micro-switch 1300 are provided on the lower side of the hold member 300 so as to face the chip mounting part 310.

In the chip mounted condition, the pushing mechanism 700 pushes the chip 1 to position the chip 1 relative to the hold member 300. The pushing mechanism 700 is located so as to face the measuring means 900 which will be described later, through the chip mounting part 310.

The pushing mechanism 700 is disposed in a hole part 340 communicated with the chip mountina part 310 of the hold member 300, and is composed of a plunger 720 and a spring (biasing member) 730 for biasing the plunger 720 upwards.

At an outer peripheral part of an intermediate part of the plunger 720, a flange 740 serving as a spring seat is projectingly formed. A tip end portion (upper end portion) of the plunger 720 is so configured as to be inserted in a recessed part 36 of the chip 1 which will be described later, in the chip mounted condition, whereby the chip 1 is favorably pushed toward the measuring means 900 side.

In addition, a lid member 360 is fixed to the hold member 300 by screws 360a and 360b, so as to seal the hold part 340.

The spring 730 is set in a compressed state, and both ends thereof abut respectively on the inside surface of the lid member 360 and the plunger 740, whereby the plunger 720 is biased upwards by the spring 730.

Incidentally, while the plunger 720 is thus biased by the spring 730, the flange 740 is engaged with a step part 370 formed at the hole part 340 and, therefore, the plunger 720 is prevented from penetrating further into the chip mounting part 310.

By the pushing mechanism 700 as above, the chip 1 is positioned relative to the hold member 300 (component measuring instrument 100) in the chip mounted condition.

The micro-switch 1300 is for detecting whether the chip 1 is mounted in the chip mounting part 310 or not.

The micro-switch 1300 is disposed in a hole part 380 communicated to the chip mounting part 310 of the hold member 300, and the hole part 380 is sealed by the structure in which the lid member 390 is fixed to the hold member 300 by the screws 390a, 390b.

In addition, the ejecting mechanism 800 is provided in the inside, on the tip end side, of the hold member 300 (in the vicinity of the opening 230 in the main frame 200). The ejecting mechanism 800 has the function of discharging the chip 1 from the component measuring instrument 100, and is composed of an eject pin 810 capable of moving toward the tip end, and a lever (not shown) for moving the eject pin 810 toward the tip end.

In the chip mounted condition, the eject pin 810 is in the inside of the hole member 300, and its tip end portion is in contact with a flange 39 of the chip 1 which will be described later (see Fig. 11). With the lever slid from this condition, the eject pin 810 is moved in the inside of the hold member 300 toward the tip end, and pushes the flange 39 toward the tip end. By this, the chip 1 is moved relative to the component measuring instrument 100 toward the tip end, and is disengaged from the chip mounting part 310 (the component measuring instrument 100).

Besides, another configuration example may be adopted in which, for example, an eccentric cam having a rotational axis on the tip end side relative to the hold member 300 (in the vicinity of the opening 230 in the main frame 200) is disposed, and the eccentric cam is rotated so as to push the flange 39 of the chip 1 toward the tip end.

In addition, the puncturing means 500 is contained and held in the inside of the hold member 300. In other words, the puncturing means 500 is mounted to the main frame 200 of the component measuring instrument 100 through the hold member 300. Therefore, the hold member 300 can be called a mounting member (structural member) for mounting the puncturing means 500 to the main frame 200.

The puncturing means 500 is for operating a puncture needle 5 (needle body 51) which will be described later, so as to puncture a cuticle with a needle tip 511, and has a plunger 510 and a spring (biasing member) 520 for biasing the plunger 510 in the direction of the tip end.

The plunger 510 is rod-like as a whole, and has a plunger main body 514 and a pair of arm parts 512. The arm parts 512 are each formed at a tip end portion of the plunger main body 514 integrally with the plunger main body 514.

The plunger main body 514 is passed through a support part 580, and is set movable within a predetermined range in its longitudinal direction.

The arm parts 512 are each provided with a recessed part 513 in the inside surface of a tip end portion thereof. A connection part 524 of the puncture needle 5 which will be described later is disengageably fitted in the recessed part 513, whereby the puncture needle 5 is connected to the puncturing means 500 in the chip mounted condition. In other words, tip end portions of the arm parts 512 constitute a holder part 530 for connecting and holding the puncture needle 5.

In addition, at an intermediate part in the longitudinal direction of the plunger 510 (in the vicinity of a boundary part between the plunger main body 514 and the arm part 512), a flange 540 serving as a spring seat is projectingly formed.

In the chip mounted condition, the spring 520 is in a compressed state, and both ends thereof abut on the flange 540 and a part (not shown) of the hold member 300, whereby the plunger 510 is biased in the direction of the tip end.

This condition is maintained by the locking of an elastically deformable elastic piece 550 to the flange 540 (see Fig. 11). The elastic piece 550 has one end portion 551 fixed (firmly attached) to the hold member 300, and has the other end portion serving as a lock part 552 for locking to the flange 540; with the one end portion 551 as a fulcrum (fixed end), the lock part 552 is displaced toward and away from the plunger 510 (as indicated by two-dotted chain lines in Fig. 11).

Besides, in this condition, a space 560 is formed between the elastic piece 550 and the hold member 300. When an unlocking member 571 shown in Fig. 10 is inserted into the space 560, the elastic piece 550 is elastically deformed so that the lock part 552 is spaced from the plunger 510. As a result, the locking of the flange 540 by the elastic piece 550 is canceled, and the plunger 510 is pushed by the spring 520 to be thereby moved in the direction of the tip end (see Fig. 12).

As shown in Fig. 10, the unlocking member 571 is formed as one body with a plate member 570 which is cantilever supported relative to the hold member 300, with its one end portion 572 as a fixed end and with its other end portion as a movable end. On the side of the other end portion of the plate member 570, a pushing part 573 is formed at a position corresponding to the operation button 260.

In addition, a spring 574 is provided between the pushing part 573 and the hold member 300.

With the operation button 260 depressed, the pushing part 573 of the plate member 570 is pushed downwards, and, attendant on this, the unlocking member 571 is moved downwards to be inserted into the space 560. In this instance, the spring 574 is set into the compressed state, to bias the operation button 260 upwards through the pushing part 573 of the plate member 570. Therefore, when the pressing on the operation button 260 is canceled, the operation button 260 is pushed upwards by the spring 574 through the pushing part 573, to be moved substantially back into its original position.

The component measuring instrument 100 as above is used with the chip (the humor sampling implement according to the present invention) 1 mounted thereto. Now, the chip (the humor sampling implement according to the present invention) 1 will be described in detail below.

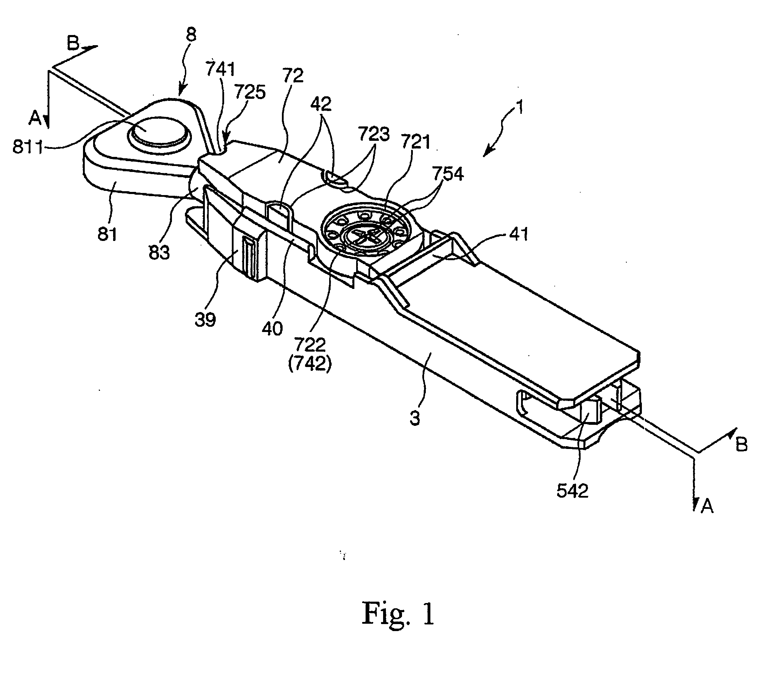

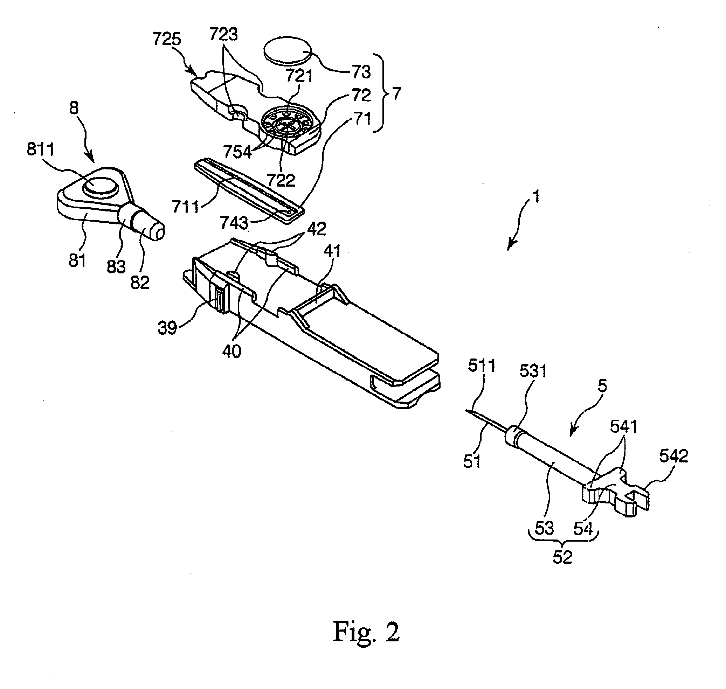

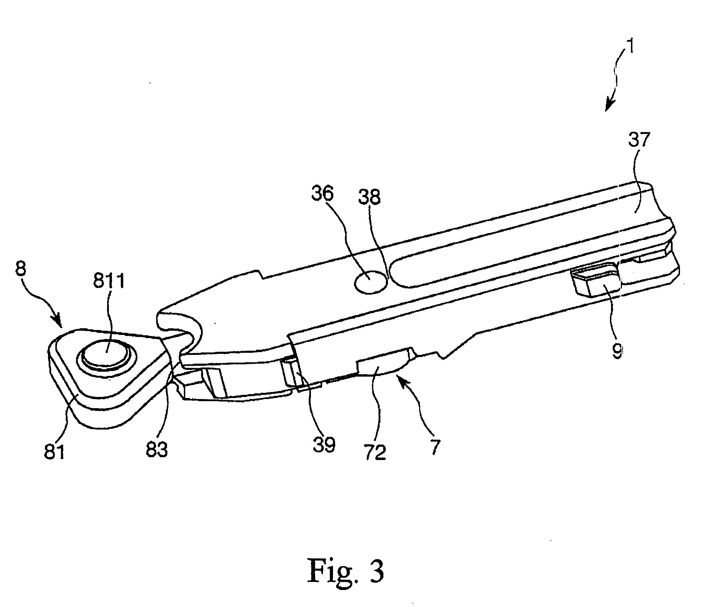

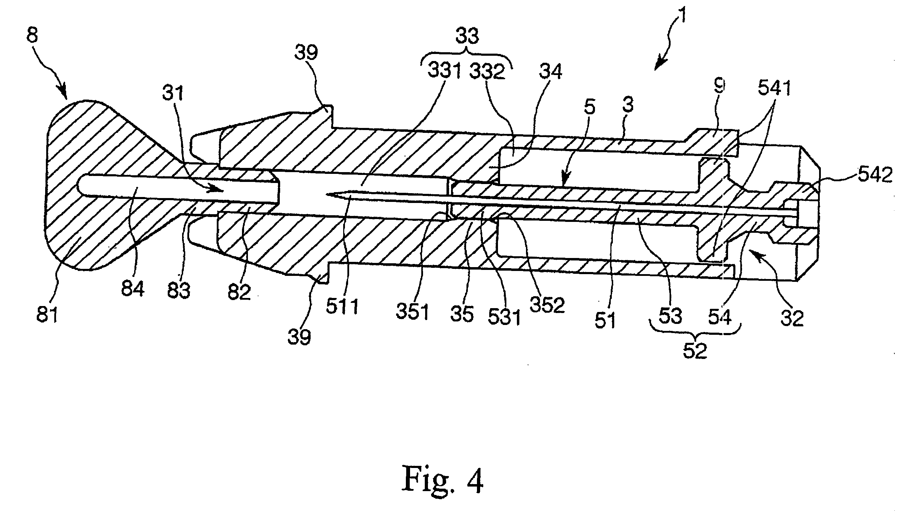

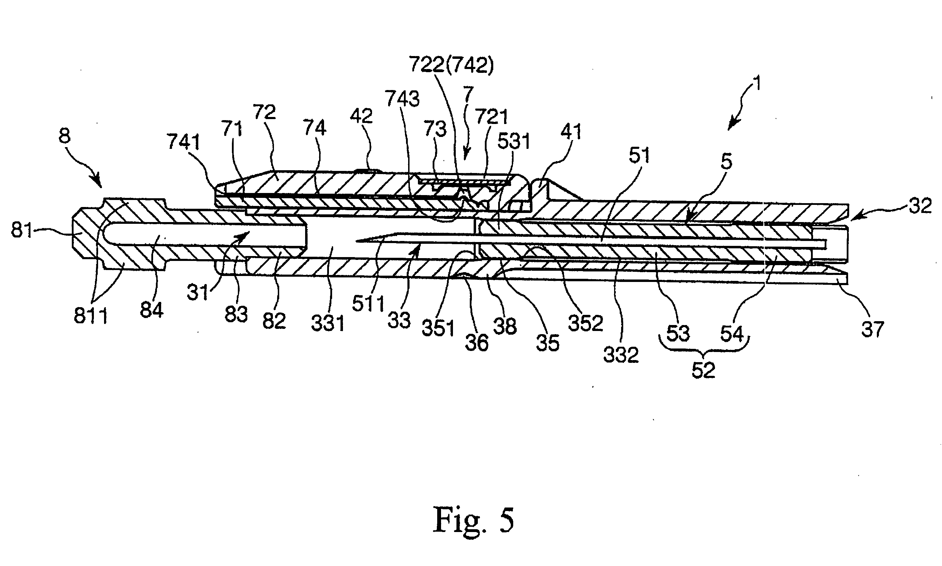

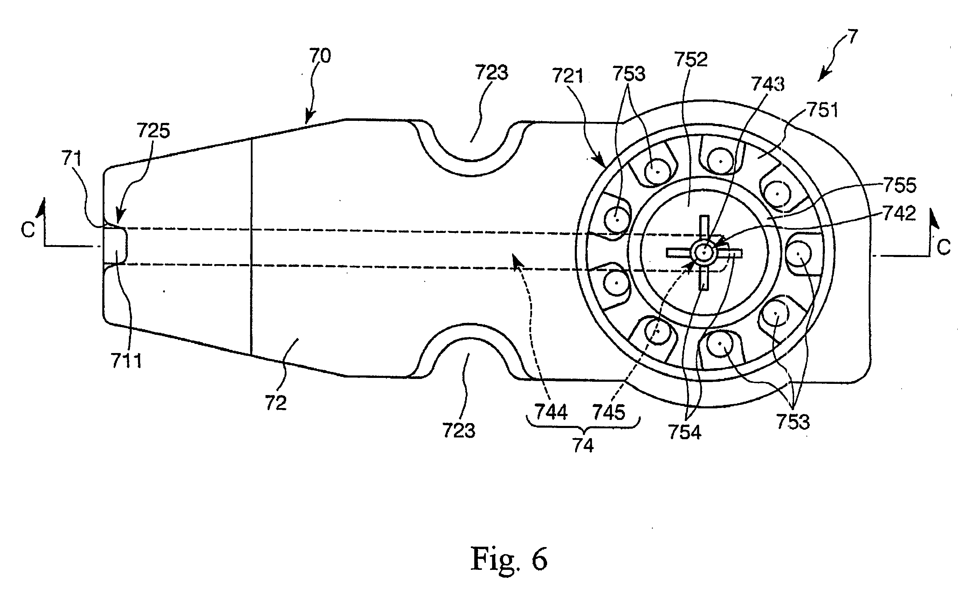

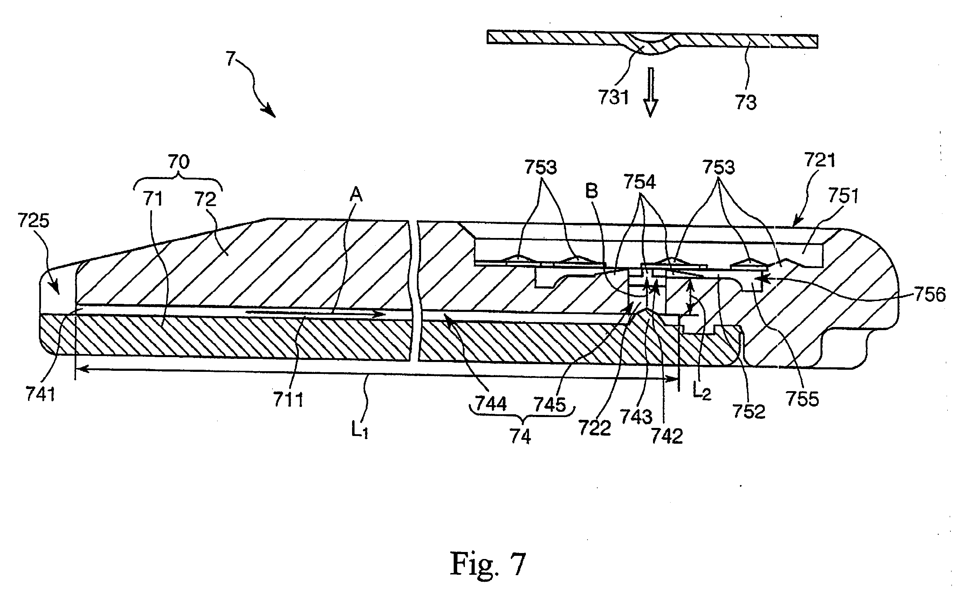

Fig. 1 is a oblique perspective view of one embodiment of the chip (the humor sampling implement according to the present invention), Fig. 2 is an exploded perspective view of the chip shown in Fig. 1, Fig. 3 is a oblique perspective view as viewed from the lower side of the chip shown in Fig. 1, Fig. 4 is a sectional view along line A-A of Fig. 1, Fig. 5 is a sectional view along line B-B of Fig. 1, Fig. 6 is a plan view of a detection unit possessed by the chip shown in Fig. 1, and Fig. 7 is a sectional view along line C-C of Fig. 6. Incidentally, in Figs. 1 to 7, the left side will be referred to as "the tip end", and the right side as "the base end"; in Figs. 1, 2, 5 and 7, the upper side will be referred to as "upper" or "upper side", and the lower side as "lower" or "lower side"; and in Fig. 3, the upper side will be referred to as "lower" or "lower side", and the lower side as "upper" or "upper side". In Fig. 1 and Fig. 6, the test paper is omitted.

The chip 1 shown in the figures has a casing 3 in which the puncture needle 5 is contained, and a detection unit 7 to which the test paper (detection part) 73 is firmly attached (fixed). Now, the component elements will be sequentially described below.

The puncture needle 5 is composed of a needle body 51, and a hub 52 firmly attached (fixed) to the needle body 51.

The needle body 51 is composed of a hollow member or solid member formed of a metallic material, for example, stainless steel, aluminum, aluminum alloy, titanium, titanium alloy, or the like, and is provided at its tip end with a sharp needle tip (cutting edge) 511. The surface (skin) of a fingertip is punctured with the needle tip 511, to cause blood (humor) to flow out of (bleed from) the puncture site.

The hub 52 is fixed (firmly attached) to the needle body 51 by, for example, fusing, adhesion with an adhesive, fitting, caulking or the like so that the needle tip 511 protrudes.

The hub 52 is composed of a cylindrical part 53 roughly cylindrical in shape on the tip end side, and a rectangular parallelepiped part 54 roughly rectangular parallelepiped in shape on the base end side. The outside diameter (diameter) of the cylindrical part 53 and the height of the rectangular parallelepiped part 54 are set approximately equal to each other.

The cylindrical part 53 is provided at its tip end portion with a fitting part 531 enlarged in diameter relative to the outside diameter of the cylindrical part 53. The fitting part 531 is fitted into a fitting part 35 of the casing 3 which will be described later.

The rectangular parallelepiped part 54 is provided at its tip end portion with a pair of projected part 541 projected sideways. The projected parts 541 each abut on a stepped part 34 of the casing 3 which will be described later.

In addition, the rectangular parallelepiped part 54 is provided at its base end portion with a connection part 542 having a shape corresponding to the shape of the holder part 530 of the plunger 510 described above. In the chip mounted condition, the connection part 542 is fitted in the holder part 530, whereby the puncture needle 5 is connected to the plunger 510 (the puncturing means 500).

The puncture needle 5 as above is movably provided in a lumen part 33 possessed by the casing 3. The casing 3 is composed of a roughly rectangular parallelepiped member, and is provided with a tip end opening 31 and a base end opening 32 for opening the lumen part 33 to the exterior, respectively at the tip end and the base end thereof. The needle body 51 possessed by the puncture needle 5 passes through the tip end opening 31 to protrude from the tip end of the casing 3 (chip 1) (see Fig. 12).

As shown in Fig. 4, the lumen part 33 is composed of a first lumen part 331 on the tip end side and a second lumen part 332 on the base end side.

The first lumen part 331 is roughly cylindrical in shape, and its cross-sectional area is set to be approximately equal to or a little greater than the cross-sectional area (maximum) of the fitting part 531 of the hub 52. In addition, the second lumen part 332 is roughly rectangular parallelepiped in shape, and its cross-sectional area is set to be approximately equal to or a little greater than the cross-sectional area (maximum) of the rectangular parallelepiped part 54 (the part of the projected parts 541).

When the puncture needle 5 is moved relative to the casing 3, the fitting part 531 of the hub 52 is moved along the inside surface of the first lumen part 331, whereas the part of the projected parts 541 of the hub 52 is moved along the inside surface of the second lumen part 332. In this instance, the fitting part 531 and the projected portions 541 serve as support parts.

In such a configuration, when the puncture needle 5 is moved relative to the casing 3, the puncture needle 5 is supported on the casing 3 at two positions of the fitting part 531 and the projected parts 541 of the hub 52, i.e., at two positions in the longitudinal direction of the puncture needle 5. Therefore, the puncture needle 5 can be moved smoothly relative to the casing 3, is prevented from unintentional shifting relative to the casing 3, and is moved in the direction of the tip end with high rectilinearity of movement. Accordingly, it is possible to favorably prevent an increase in the pain of the patient attendant on unintentional shifting of the needle tip 511 of the needle body 51.

In addition, since the shape of the first lumen part 331 and the shape of the second lumen part 332 are different from each other, the casing 3 has the stepped part 34 formed at the boundary part between the two lumen parts. Therefore, when the puncture needle 5 is moved in the direction of the tip end, the projected parts 541 of the hub 52 come into contact with the stepped part 34. As a result of the abutment, the movement of the puncture needle 5 is stopped, and the length of protrusion of the needle tip 511 of the puncture needle 5 from the casing 3 is restricted. Namely, in this embodiment, the stepped part 34 formed in the casing 3 and the projected parts 541 formed on the hub 52 of the puncture needle 5 and abutting on the stepped part 34 constitute a protrusion length restricting means. With the protrusion length restricting means thus provided, puncturing of a finger (humor sampling site) to an excessively large depth can be prevented from occurring.

Further, since the fitting part 531 and the projected parts 541 serving as support parts make only partial contact with the casing 3, the frictional resistance at the time of movement of the puncture needle 5 relative to the casing 3 is small, and the movement proceeds smoothly. Accordingly, it is easy to control the movement of the puncture needle 5 relative to the casing 3.

In addition, the first lumen part 331 is provided at its base end portion with a fitting part 35 reduced in diameter as compared with the inside diameter of the first lumen part 331. The fitting part 531 of the puncture needle 5 is fitted in the fitting part 35. By this fitting, the puncture needle 5 is fixed relative to the casing 3.

The fitting force (fixing force) between the fitting part 35 and the fitting part 531 is set to be greater than the force required for connecting the connection part 542 of the puncture needle 5 to the holder part 530 of the puncturing means 500 (the plunger 510). This ensures that the puncture needle 5 can be connected to the puncturing means 500 without any trouble.

Besides, the force required for fitting the fitting part 531 in the fitting part 35 is set to be a little greater than the force required for canceling the connection between the connection part 542 of the puncture needle 5 and the holder part 530 of the puncturing means 500. This setting produces the following functions or effects.

In the chip 1 after use, the fitting part 531 of the puncture needle 5 is located on the tip end side relative to the fitting part 35 of the casing 3. When the casing 3 is moved in the direction of the tip end from this condition in order to disengage the chip 1 from the component measuring instrument 100, the puncture needle 5 connected to the puncturing means 500 is relatively moved toward the base end side, whereby the fitting part 531 is fitted into the fitting part 35. Substantially simultaneously with this fitting or before or after the fitting, the connection between the connection part 542 of the puncture needle 5 and the holder part 530 of the puncturing means 500 is released, and the chip 1 is disengaged from the component measuring instrument 100. In the chip 1 thus disengaged from the component measuring instrument 100, the fitting part 531 is fitted in the fitting part 35 either substantially wholly or partly, and the puncture needle 5 is fixed relative to the casing 3. Therefore, even where the tip end of the chip 1 is directed vertically down, the needle tip 511 of the puncture needle 5 is prevented from protruding from the tip end of the casing 3, and, even where the base end of the chip 1 is directed vertically down, the puncture needle 5 is prevented from slipping off from the casing 3. Thus, dangers such as mistaken damaging of a skin or the like, scattering of blood with the result of contamination of the surroundings, etc. can be prevented, and high safety can be secured.

The fitting part 35 of the casing 3 is provided with tapered parts 351 and 352 at its tip end portion and base end portion, respectively.

In the assembling step of the chip 1, the puncture needle 5 is inserted via the base end opening 32 of the casing 3, and the fitting part 531 of the hub 52 is fitted into the fitting part 35 of the casing 3; in this case, since the tapered part 352 is formed at the base end portion of the fitting part 35, the fitting operation can be easily performed.

On the other hand, since the tapered part 351 is provided at the tip end portion of the fitting part 35, the fitting of the fitting part 531 of the hub 52 into the fitting part 35 of the casing 3 can be carried out more easily and securely, at the time of disengaging the chip 1 from the component measuring instrument 100 as above-mentioned.

In addition, the lower surface of the casing 3 is provided with a recessed part 36 and a guide groove 37 in recessed or sunken forms.

The recessed part 36 is a part in which a tip end portion of the plunger 720 (pushing mechanism 700) is inserted in the chip mounted condition, and the recessed part 36 is formed to have a shape corresponding to the shape of the tip end portion of the plunger 720.

The guide groove 37 is formed along the longitudinal direction of the casing 3, from the base end of the casing 3 to the vicinity of the recessed part 36. The guide groove 37 has the function of guiding the tip end portion of the plunger 720 (pushing mechanism 700) to the recessed part 36. With the guide groove 37 provided, the tip end portion of the plunger 720 can be guided to the recessed part 36 more smoothly and securely.

The cross-sectional shape of the guide groove 37 is set to correspond to the longitudinal sectional shape of the tip end portion of the plunger 720.

In addition, the guide groove 37 may be formed in continuity with the recessed part 36; in this embodiment, however, they are not in continuity, and a bank part 38 is formed between the guide groove 37 and the recessed part 36.

In this configuration, at the time of mounting the chip 1 to the component measuring instrument 100, the tip end portion of the plunger 720 is moved toward the recessed part 36 while being guided by the guide groove 37, and rides over the bank part 38, to reach the inside of the recessed part 36. In this instance, a click feeling is obtained, so that the secure mounting of the chip 1 into the chip mounting part 310 can be recognized, which is convenient.

Besides, the casing 3 has a pair of flanges 39 projectingly formed on both side surfaces of a tip end portion thereof. In the chip mounted condition, the flanges 39 each abut on the tip end of the main frame 200 of the component measuring instrument 100. At the time of disengaging the chip 1 from the component measuring instrument 100, the eject pins 810 are each moved in the direction of the tip end, and their tip ends abut on the flanges 39, to push the flanges 39 in the direction of the tip end. By this, the chip 1 is moved relative to the component measuring instrument 100 in the direction of the tip end, and is disengaged from the chip mounting part 310 (the component measuring instrument 100).

In addition, the upper surface of the casing 3 is provided with a pair of opposed wall parts 40, a wall part 41, and projections 42. The wall parts 40 are erected along both side parts on the tip end side of the casing 3, and the wall part 41 is erected at an intermediate part in the longitudinal direction of the casing 3, substantially orthogonally to the longitudinal direction. The detection unit 7 which will be described later is mounted into the region surrounded by the wall parts 40 and 41. Namely, the part of the region constitutes a detection unit mounting part for mounting the detection unit 7 therein.

On the inside of the wall parts 40, a pair of the projections 42 are erected in contact with the wall parts 40. The projections 42 are inserted into recessed parts 723 formed in a cover 72 of the detection unit 7 respectively, whereby the detection unit 7 is positioned and fixed relative to the chip 1. Incidentally, in this condition, the tip end position of the detection unit 7 and the tip end position of the casing 3 substantially coincide with each other.

The detection unit 7 is for detecting the glucose (predetermined component) in blood (humor).

As shown in Figs. 6 and 7, the detection unit 7 has a main frame part 70, and a test paper (detection part) 73 provided in the main frame part 70.

The main frame part 70 constitutes a part which supports the test paper 73 and which serves for mounting of the detection unit 7 to the casing 3. The main frame part 70 is composed of a base (lower member) 71, and a cover (upper member) 72 piled on the base 71.

The base 71 is composed of a flat plate-like member. The base 71 is provided with a groove 711 opening to the upper side. The groove 711 is formed in a roughly rectilinear shape and along the longitudinal direction of the base 71. In addition, the groove 711 is opened at the tip end of the base 71.

The cover 72 is composed of a roughly regular parallelepiped member. The lower surface of the cover 72 is provided with a recessed part 724 along the longitudinal direction thereof. The base 71 is firmly attached (fixed) to the inside of the recessed part 724.

The upper surface of the cover 72 is provided at its base end portion with a test paper placing part 721 on which to place the test paper 73. The test paper placing part 721 is composed of a recessed part which is roughly circular (plane shape corresponding to the test paper 73) in plan view, and the bottom surface thereof is provided in its central area with a through-hole 722 communicated with the recessed part 724.

In the condition where the base 71 is firmly attached to the inside of the recessed part 724 of the cover 72, the space formed (defined) therebetween and the through-hole 722 formed in the cover 72 constitute a blood transfer channel (humor transfer channel) 74 for transferring blood (humor).

Examples of the method for firm attachment between the base 71 and the cover 72 include fusing (heat fusing, ultrasonic fusing, high-frequency fusing), pressure sensitive adhesion, adhesion with an adhesive, etc.

In addition, the cover 72 is provided with a pair of recessed parts 723 in both side surfaces thereof. The projections 42 of the casing 3 are inserted in the recessed parts 723, whereby the detection unit 7 is positioned and fixed relative to the chip 1.

Besides, the cover 72 is provided with a recessed part at its tip end, and, at this recessed part, a tip end portion of the groove 711 formed in the base 71 is exposed to the exterior of the detection unit 7. This recessed part constitutes a blood point adhesion part 725 to which to adhere the blood being protuberant on the cuticle upon puncture. With the blood adhered to the blood point adhesion part 725, the blood is introduced into the blood transfer channel 74 efficiently.

The test paper placing part 721 is composed of a recessed part 751 for containing the test paper 73, and a recessed part 752 formed on the lower side of the recessed part 751 and smaller in diameter than the recessed part 751. The recessed part 752 is provided with the above-mentioned through-hole 722 in its bottom surface. The recessed part 751 has an upper edge part being tapered, and has a plurality of pedestal parts 753 erected on the bottom surface thereof so as to surround the periphery of the recessed part 752.

In this embodiment, the pedestal parts 753 are roughly conical, and nine pedestal parts 753 are provided at roughly equal intervals along the periphery of the recessed part 752. In the condition where the test paper 73 is placed on the test paper placing part 721, each of the pedestal parts 753 supports a peripheral part of the test paper 73 with its part near its apex part.

In addition, the bottom surface of the recessed part 752 is provided, along an opening (blood outflow port 742) of the through-hole 722, with a plurality of pedestal parts 754 and with an annular (ring-shaped) groove 755 at the boundary part between itself and the recessed part 751.

The pedestal parts 754 are composed of small pieces provided in the shape of a cross intersecting at the opening of the through-hole 722, and are tapered so that their height is gradually reduced outwards.

The pedestal parts 754 have the function of supporting the test paper 73 together with the pedestal parts 753, and their parts near their apex parts support a part near a central part of the test paper 73 (a projected part 731 of the test paper 73 which will be described later).

In the configuration as above, in the condition where the test paper 73 is placed on the test paper placing part 721, a comparatively large gap 756 is formed (defined) between the lower surface of the test paper 73 and the upper surface of the test paper placing part 721 (particularly, the recessed part 752). The gap 756 is communicated with the blood transfer channel 74 via the spaces between the pedestal parts 754.

The gap 756 as above functions as an air vent for the blood transfer channel 74, and the pneumatic pressure prevents the sampled blood from stagnating in the course of the blood transfer channel 74.

In addition, the gap 756 also has the function of assisting (accelerating) the development of blood on the test paper 73. Specifically, the blood flowing out of the through-hole 722 (the blood transfer channel 74) is supplied to the test paper 73 while spreading radially in the gap 756, so that the development of the blood on the test paper 73 occurs more speedily and uniformly.

The blood transfer channel 74 has a blood inflow port 741 opening to the tip end of the detection unit 7, and the blood outflow port 742 opening to the upper side of the detection unit 7.

In addition, the blood transfer channel 74 in this embodiment is composed of a first blood transfer channel (first humor transfer channel) 744 formed (defined) by the base 71 and the cover 72, and a second blood transfer channel (second humor transfer channel) 745 continuous with the first blood transfer channel 744 and composed of the through-hole 722.

With this configuration, i.e., with the configuration in which a part of the humor transfer channel is defined by the base (lower member) 71 and the cover (upper member) 72, the blood transfer channel 74 having a large overall length and a small sectional area can be comparatively easily formed through a simple method, as compared with the case where, for example, a fine hole part is bored in a block-like member to form a blood transfer channel 74 as a whole.

Besides, the first blood transfer channel 744 opens to the blood inflow port 741 and extends along the longitudinal direction of the detection unit 7, whereas the second blood transfer channel 745 extends along the thickness direction of the detection unit 7 and opens at the blood outflow port 742. In other words, the blood transfer direction in the first blood transfer channel 744 (direction A in Fig. 7) and the blood transfer direction in the second blood transfer channel 745 (direction B in Fig. 7) are substantially orthogonal to each other. In addition, the blood outflow port 742 opens at roughly the center of the test paper placing part 721 (the test paper 73).

The blood contacting the blood point adhesion part 725 is introduced via the blood inflow port 741 into the first blood transfer channel 744, and is transferred in the first blood transfer channel 744 by capillarity. Next, the blood reaching the boundary part between the first blood transfer channel 744 and the second blood transfer channel 745 is changed in transfer direction by about 90° so as to be along the inside wall surface of the second blood transfer channel 745, and is transferred in the second blood transfer channel 745 up to the blood outflow port 742 in a drawn-up manner by the capillarity in the second blood transfer channel 745. Then, the blood flowing out of the blood outflow port 742 is supplied to the test paper 73 while spreading radially in the gap 756.

Incidentally, in the following description, the blood transfer direction in the blood transfer channel 74 will be referred to simply as "the transfer direction", the section along the direction parallel to the transfer direction in the blood transfer channel 74 will be referred to as "the longitudinal section", and the section along the direction perpendicular to the transfer direction will be referred to as "the cross-section".

In the blood transfer channel 74 as above, the shapes and sizes of respective parts and the like are preferably set as follows. Now, the shapes and sizes of the first blood transfer channel 744 and the second blood transfer channel 745 and the like will be described below.

The cross-sectional area (average) of the first blood transfer channel 744 is not particularly limited, and is preferably in the range of about 0.05 to 30 mm², more preferably about 0.1 to 10 mm². If the cross-sectional area (average) of the first blood transfer channel 744 is too small, the transfer of blood by capillarity (hereinafter referred to simply as "the blood transfer") is slow, and a long time is needed to obtain a sufficient amount of blood. On the other hand, if the cross-sectional area (average) of the first blood transfer channel 744 is too large, it is difficult to achieve the blood transfer.

The cross-sectional shape of the first blood transfer channel 744 may be any shape, for example, tetragon such as rectangle, square, rhombus, etc., triangle, hexagon, octagon, circle, ellipse, or the like; however, the shape is preferably a rectangle (as shown in Fig. 2, the cross-sectional shape of the groove 711 is angular U-shaped). This ensures that the amount of blood remaining in the first blood transfer channel 744 can be reduced.

From this point of view, the cross-sectional shape of the first blood transfer channel 744 is particularly preferably a thin type (small-height) rectangle; in this case, the height is preferably about 0.05 to 0.5 mm, and the width is preferably about 0.5 to 3 mm, more preferably about 0.5 to 1 mm.

In addition, the length of the first blood transfer channel 744 (overall length: L₁ in Fig. 7) is appropriately set according to the cross-sectional area (average) of the first blood transfer channel 744, and is not particularly limited; however, the length is preferably about 1 to 25 mm, more preferably about 5 to 20 mm.

On the other hand, the cross-sectional area (average) of the second blood transfer channel 745 is also preferably in the range of about 0.05 to 30 mm², more preferably about 0.1 to 10 mm², by the same reason as described above for the first blood transfer channel 744.

In addition, the cross-sectional shape of the second blood transfer channel 745 is also not particularly limited, and may be any shape, like the cross-sectional shape of the first blood transfer channel 744.

In this embodiment, the cross-sectional shape of the second blood transfer channel 745 is roughly the same as the bottom part shape of a convex part 743 which will be described later. Specifically, the cross-sectional shape is roughly circular, as shown in Fig. 6.

Besides, the cross-sectional area of the second blood transfer channel 745 is set to be approximately equal to the area of the bottom part of the convex part 743, as shown in Fig. 7, and the cross-sectional area is substantially constant along the transfer direction. Namely, the second blood transfer channel 745 is in the form of a straight pipe. This configuration makes it possible to realize an efficient blood transfer (upward drawing of blood) even in the second blood transfer channel 745 in which the blood transfer against gravity is performed.

The length of the second blood transfer channel 745 (overall length: L₂ in Fig. 7) is appropriately set according to the cross-sectional area (average) of the second blood transfer channel 745, and is not particularly limited; however, the length is preferably in the range of about 0.1 to 1.0 mm, more preferably about 0.4 to 0.8 mm.

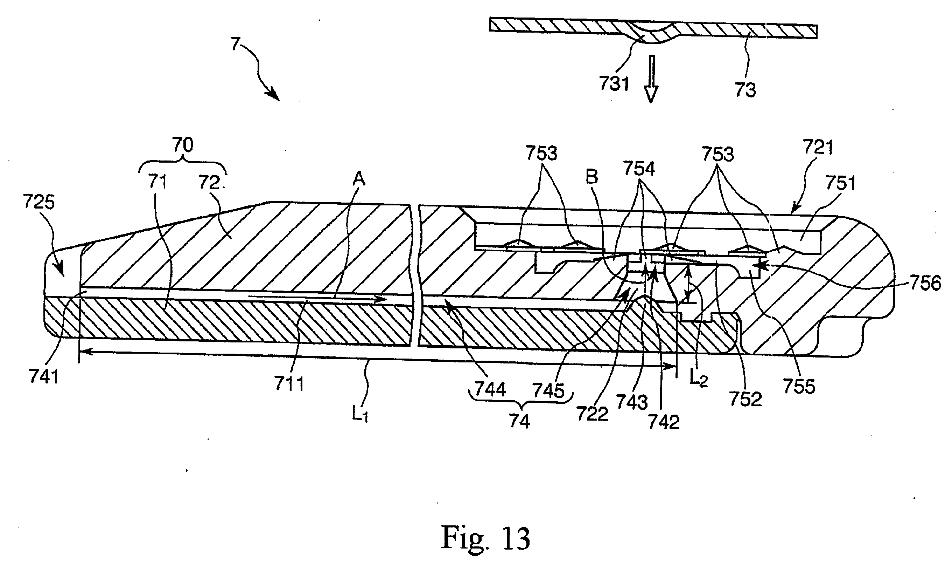

Incidentally, the second blood transfer channel 745 may be configured as shown in Fig. 13. Fig. 13 shows another configuration example of the second blood transfer channel (second humor transfer channel).

The second blood transfer channel 745 shown in Fig. 13 is so shaped that its cross-sectional area is gradually reduced toward a blood outflow port 742, and constitutes a cross-sectional area gradually decreasing part. This structure accelerates the blood transfer, and make it possible to achieve a more efficient blood transfer.

In this case, R₁/R₂ is preferably in the range of from 0.3 to 0.8, more preferably from 0.4 to 0.7, where R₁ [mm²] is the minimum cross-sectional area of the second blood transfer channel 745, and R₂ [mm²] is the maximum cross-sectional area of the channel. This ensures that the accelerating effect on the blood transfer in the second blood transfer channel 745 is displayed more conspicuously.

Meanwhile, in the present invention, the main frame part 70 of the detection unit 7 is provided with a convex part 743 arranged to be in overlapped relationship with the test paper 73 (be directly under the test paper 73) in plan view and protruding in the blood transfer channel 74 toward the blood outflow port 742 (test paper 73).

In this embodiment, the convex part 743 is provided at the bottom surface of an end portion on the humor outflow port 742 side of the first blood transfer channel 744 of the main frame part 70 (base 71) (at the boundary part between the first blood transfer channel 744 and the second blood transfer part 745) so as to protrude in the second blood transfer channel 745.

With the convex part 743 thus provided, the cross-sectional area of the blood transfer channel 74 on the blood outflow port 742 side can be prevented from increasing, or the cross-sectional area can be reduced. As a result, the efficiency of the blood transfer can be prevented from being lowered, or can be increased. In addition, while the transfer of blood from the first blood transfer channel 744 into the second blood transfer channel 745 occurs after the space at the boundary part between the first blood transfer channel 744 and the second blood transfer channel 745 is sufficiently filled with the blood, the provision of the convex part 743 ensures that the inside volume of the space at the boundary part between the first blood transfer channel 744 and the second blood transfer channel 745 is reduced, and the transfer is achieved speedily. Further, it is also possible to favorably prevent or suppress the generation of meniscus in the vicinity of the convex part 743. These points ensure that the collected blood can be transferred to the test paper 73 more securely and speedily.

Therefore, measurement of blood sugar level can be efficiently performed, without such troubles as the trouble that the blood stagnates in the blood transfer channel 74 and the chip 1 should be discarded wastefully or the patient should be subjected to blood sampling again.

Here, V₁/V₂ is preferably in the range of from 0.04 to 0.7, more preferably from 0.05 to 0.5, where V₁ [mm³] is the volume of the convex part 743, and V₂ [mm³] is the inside volume of the second blood transfer channel 745. With V_{1/}V₂ in the range, the above-mentioned effect can be further enhanced.

The convex part 743 is provided at a position corresponding to the blood outflow port 742 of the blood transfer channel 74, i.e., at a position corresponding roughly to the center of the test paper 73. This ensures that the transfer and supply of the blood to the test paper 73 are achieved more smoothly.

The shape of the convex part 743 may be any shape, and is preferably such a shape that the cross-sectional area is reduced toward the upper side (for example, a artillery projectile-like shape or the like), as shown in Fig. 7. This ensures that the change in the transfer direction from the first blood transfer channel 744 to the second blood transfer channel 745 is also achieved favorably.

In addition, where the shape of the convex part 743 is different from the above-mentioned shape, examples of the shape (longitudinal sectional shape) include tetragon such as square, rhombus, trapezoid, etc., triangle, hexagon, octagon, circle, ellipse, and the like.

The convex part 743 preferably has a surface treated to be hydrophilic. This ensures that the blood can be transferred toward the blood outflow port 742 of the blood transfer channel 74 more speedily.

The treatment for rendering the surface hydrophilic can be carried out by a physical activating treatment such as plasma treatment, glow discharge, corona discharge, irradiation with UV rays, etc., or application of (coating with) surfactant, water-soluble silicone, hydroxypropyl cellulose, polyethylene glycol, polypropylene glycol, or the like.

From these points of view, it is preferable that the inside surfaces of the first blood transfer channel 744 and the second blood transfer channel 745 are also preliminarily treated to be hydrophilic.

Examples of the method for obtaining the convex part 743 and the base 71 as above include (I) a method of integrally molding them by injection molding, (II) a method of applying etching to a parent material to obtain predetermined shapes, (III) a method of forming the surface of a flat plate-like parent material into predetermined shapes by use of a printing process, (IV) a method of firmly attaching (fixing) a predetermined shaped member to the surface of a flat plate-like parent material, etc. According to the methods of (I) and (II), the convex part 743 and the base 71 high in dimensional accuracy can be obtained easily. Besides, according to the methods of (III) and (IV), it is possible to contrive a reduction in the production cost of the convex part 743 and the base 71. Incidentally, the methods of (I) to (IV) may be used in combination of arbitrary two or more of them.

At the test paper placing part 721 as above-mentioned, the test paper 73 is firmly attached (fixed) to the pedestal parts 753 and the pedestal parts 754 by, for example, fusing, adhesion with an adhesive, or the like method.

The test paper 73 is capable of detecting glucose contained in the blood transferred through the blood transfer channel 74, and, for example, has a reagent (coloration reagent) carried on (impregnating) a carrier (absorber) capable of absorbing the blood. The carrier is preferably composed of a porous film. In this case, the porous film preferably has such a pore diameter that erythrocytes in the blood can be filtered.

The use of the carrier composed of the porous film ensures that, in the case where the reagent for impregnation is particularly a reagent system including the process of reaction with oxygen as a substrate such as an oxidase reaction, even in the condition where the blood accepting side is covered with blood after development of the blood on the test paper 73, oxygen in the atmospheric air is supplied from the reaction side (opposite side), so that the reaction can be made to proceed swiftly and, therefore, the colored condition can be detected without removing the blood.

Other than the porous film, examples of the carrier of the test paper 73 include sheet form porous base materials such as non-woven fabric, woven fabric, oriented sheet, etc.

Examples of the material constituting the carrier of the porous film or the like include polyesters, polyamides, polyolefins, polysulfones, celluloses, etc. For impregnating the carrier with an aqueous solution containing the reagent dissolved therein or for speedily performing the absorption and development of blood at the time of sampling the blood, the constituent material is preferably a hydrophilic material or a material treated to be hydrophilic by the same method as above-mentioned.

In addition, the carrier of the test paper 73 may have a single-layer sheet configuration or may have a multi-layer configuration in which a plurality of sheets are laminated.

While the shape in plan view of the carrier of the test paper 73 is roughly circular in the configuration shown in the figure, the shape may be any other shape, for example, tetragon such as rectangle, rhombus, etc., triangle, hexagon, octagon, ellipse and the like.

In the case of measurement of blood sugar level, examples of the reagent with which the carrier (porous film) is to be impregnated include glucose oxidase (GOD), peroxidase (POD), and color formers (coloring reagents) such as 4-aminoantipyrine, N-ethyl-N-(2-hydroxy-3-sulfopropyl)-m-toluidine, etc. Other than the above, according to the component to be measured, examples of the reagent include the reagents capable of reaction with a blood component (predetermined component), such as ascorbic acid oxidase, alcohol oxidase, alcohol dehydrogenase, galactose oxidase, fructose dehydrogenase, cholesterol oxidase, cholesterol dehydrogenase, lactic acid oxisase, lactic acid dehydrogenase, bilirubin oxidase, xanthin oxidase, etc., and the same color formers (coloring reagents) as above-mentioned. Besides, a buffer such as phosphoric acid buffer may further be contained. Incidentally, the kind or component of the reagent is naturally not limited to these.

In addition, the test paper 73 has a projected part 731 formed near a central part thereof. In the condition where the test paper 73 is placed on the test paper placing part 721, the projected part 731 makes contact with and is supported by the pedestal parts 754. This ensures that the test paper 73 can be stably fixed by the test paper placing part 721, and non-uniform development of blood on the test paper 73 due to deformation (curving, distortion, waving or the like) of the test paper 73 can be prevented from occurring.

A lid body 8 is mounted to a tip end portion of the chip 1 so as to close the lumen part 33 of the casing 3. The lid body 8 is mounted to the chip 1 before use (the chip 1 yet to be used), and is detached at the time of using the chip 1. The lid body 8 has a main body part 81 and a fitting part 82.

The fitting part 82 is roughly cylindrical in shape, and its outside diameter is set to be approximately equal to or a little greater than the inside diameter of a first lumen part 331 of the casing 3.

The fitting part 82 is inserted and fitted into a tip end portion of the first lumen part 331 of the casing 3. By this, the lid body 8 is mounted to the casing 3 (the chip 1). Incidentally, the fitting part 82 is tapered at an edge part of a base end portion thereof, whereby the fitting part 82 can be inserted into the first lumen part 331 of the casing 3 more easily.

Before use, the chip 1 is in the condition where the fitting part 531 of the hub 52 (the puncture needle 5) is fitted in the fitting part 35 of the casing 3, and, when the fitting part 82 of the lid body 8 is mounted to a tip end portion of the casing 3 by fitting, the seal performance of the first lumen part 331 of the casing 3, i.e., the seal performance of the lumen part 33 of the casing 3 where the needle tip 511 of the needle body 51 (the puncture needle 5) is located is secured. This prevents bacteria from penetrating into the first lumen part 331. Therefore, the sterilized condition obtained by a sterilizing treatment applied to the chip 1 is maintained until the lid body 8 is detached from the chip 1.

Here, the expression "the seal performance of the lumen part 33 is secured" means the condition where bacteria cannot substantially penetrate into the lumen part 33. Although it is preferable that gas-tightness of the lumen part 33 is secured, the gas-tightness may not necessarily be secured, and it suffices for the gas-tightness to be at such a level that the above-mentioned effect can be displayed favorably.

An enlarged diameter part 83 enlarged in diameter relative to the outside diameter of the fitting part 82 is formed between the main body part 81 and the fitting part 82. When the lid body 8 is mounted to the chip 1, the base end surface of the enlarged diameter part 83 abuts on the tip end surface of the casing 3, whereby the lid body 8 is positioned relative to the chip 1.

The main body part 81 is roughly triangular in shape in plan view, and it is a part to be gripped by fingers or the like at the time when the lid body 8 is mounted to or detached from the casing 3.

The main body part 81 is provided at its central part with a convex part 811 formed projectingly on a surface to be gripped by fingers or the like. The convex part 811 has the function of preventing slip at the time of gripping the main body part 81, i.e., it constitutes an anti-slip means. With the convex part 811 provided, the main body part 81 can be gripped by fingers or the like more assuredly, and the lid body 8 can be mounted to and detached from the casing 3 (the chip 1) more assuredly.

In addition, the lid body 8 is provided with a hole part 84 extending along the longitudinal direction thereof from the base end to an intermediate part of the main body part 81. The hole part 84 is a space capable of at least containing the needle tip 511 of the needle body 51 (the puncture needle 5), and is so formed that the center axis of the hole part 84 and the center axis of the needle body 51 coincide substantially with each other. This ensures that, even when the puncture needle 5 is unintentionally moved in the direction of the tip end (when the puncture needle 5 is erroneously jetted) in the condition where the lid body 8 is mounted to the casing 3, the needle body 51 is contained in the hole part 84 and, therefore, the needle tip 511 is prevented from being deformed or broken. Therefore, by returning to the condition where the fitting part 531 of the hub 52 is fitted in the fitting part 35 of the casing 3, the chip 1 can be again set into the unused state, so that the number of chips 1 thrown away wastefully can be reduced.

Examples of the materials constituting the hub 52, the casing 3, the base 71 (inclusive of the convex part 743), the cover 72 and the lid body 8 of the puncture needle 5 as described above include thermoplastic resins such as ABS resin, polyethylene, polypropylene, polystyrene, polyvinyl chloride, polyvinylidene chloride resin, polyphenylene oxide, thermoplastic polyurethane, polymethyl methacrylate, polyoxyethylene, fluoro-resin, polycarbonate, polyamide, acetal resin, acrylic resin, polyethylene terephthalate, etc., and thermosetting resins such as phenol resin, epoxy resin, silicone resin, unsaturated polyester, etc. Besides, for example, various ceramic materials, various metallic materials and the like may also be used as the constituent materials.

In addition, the chip 1 as above is provided with a mis-mounting preventive means for preventing the chip 1 from being set in the wrong sense at the time of mounting the chip 1 to the component measuring instrument 100. This makes it possible to favorably prevent malfunction of the component measuring instrument 100 from occurring. Now, the mis-mounting preventive means will be described in detail below.

At a base end portion of the casing 3 of the chip 1, a rib 9 is projectingly formed on one side surface (right side surface). On the other hand, in the inside surface (right inside surface) of the chip mounting part 310 of the component measuring instrument 100, a groove 311 is formed along the longitudinal direction. At the time of mounting the chip 1 to the component measuring instrument 100, the rib 9 is guided by the groove 311.

Therefore, when it is intended to mount the chip 1 to the component measuring instrument 100 in the wrong sense as to the vertical direction, the groove 311 into which the rib 9 is to be inserted is absent, so that the chip 1 cannot be mounted into (mounted to) the chip mounting part 310.

Besides, as has been described above, the wall parts 40, 41, the projections 42 and the flange 39 are projectingly formed on the tip end side of the casing 3, and the shape of the chip 1 on the side of the tip end of the casing 3 and that on the side of the base end of the casing 3 are considerably different from each other.

Therefore, when it is intended to mount the chip 1 to the component measuring instrument 100 with mistakes as to the front-rear direction and the vertical direction, the chip 1 cannot be mounted into (mounted to) the chip mounting part 310, since the shape of the chip 1 on the side of the tip end of the casing 3 and that on the side of the base end of the casing 3 differ from each other.

Thus, the mis-mounting preventive means is composed of the structure in which the shape of the chip on the side of the tip end of the casing 3 and that on the side of the base end of the casing 3 are different from each other, whereby the number of component parts and the production cost of the chip 1 can be prevented from being increased.

In addition, where the detection unit 7 is for measurement of blood sugar level as in this embodiment, the provision of the chip 1 with the mis-mounting preventive means ensures that even a patient whose sight has been conspicuously weakened (some of diabetes patients are conspicuously weakened in sight due to a complication) can mount the chip 1 correctly to the component measuring instrument 100, which is convenient.

Now, the method (function) of using the chip 1 by mounting it in the component measuring instrument 100, i.e., an example of the method of blood sampling (the method of humor sampling according to the present invention), will be described below.
[1] First, the chip 1 is inserted via the opening 230 of the main frame 200 into the chip mounting part 310 of the hold member 300, and the connection part 542 of the puncture needle 5 is fitted into the holder part 530 of the plunger 510. By this, the puncture needle 5 and the puncturing means 500 are connected to each other.
   Further, the chip 1 is pushed in the direction of the base end, whereon the plunger 510 is moved in the direction of the base end against the biasing force of the spring 520.
   Here, in the condition before the insertion of the chip 1, the flange 540 of the plunger 510 is located on the tip end side relative to the lock part 552. When the plunger 510 is moved in the direction of the base end, an edge part of the flange 540 attendantly abuts on the tip end surface (inclined surface) of the lock part 552, and pushes the tip end surface in the direction of spacing away from the plunger 510. By this, the elastic piece 550 is deflected, the lock part 552 is moved, and the flange 540 is moved toward the base end side by riding over the lock part 552.
   As a result, the flange 540 is locked on the lock part 552 even when the pushing force exerted on the plunger 510 in the direction of the base end by the chip 1 is released, so that the plunger 510 is restricted in moving in the direction of the tip end. Incidentally, in this instance, the spring 520 is set in a compressed state.
   With the chip 1 pushed in further in the direction of the base end, the base end of the flange 540 abuts on the tip end of the support part 580, whereby the plunger 510 is inhibited from moving further in the direction of the base end. By this, movement of the puncture needle 5 in the direction of the base end is also inhibited, but since the casing 3 is moved in the direction of the base end, the fitting of the fitting part 531 of the puncture needle 5 in the fitting part 35 of the casing 3 is released.
   Besides, substantially simultaneously with this, a tip end portion of the plunger 720 of the pushing mechanism 700 is inserted into the recessed part 36 of the chip 1. By this, the chip 1 is positioned to an appropriate position in the chip mounting part 310, and the position of the test paper 73 relative to the pushing mechanism 700 is also made appropriate.
   In this condition (namely, the chip mounted condition), preparation for puncturing with the puncture needle 5 and preparation for blood (specimen) sampling are completed. Thereafter, the lid body 8 mounted to the chip 1 is detached.
[2] Next, substantially simultaneously with the mounting of the chip 1, the micro-switch 1300 is turned ON, whereby each part of the component measuring instrument 100 is started, resulting in the condition where measurement is possible.
[3] Subsequently, a fingertip (finger) is put in contact with the tip end of the chip 1. In this condition, the operation button 260 is depressed, and the puncturing means 500 is operated.
   First, in conjunction with the depressing of the operation button 260, the unlocking member 571 is also moved downwards, and inserted into the space 560. By this, the lock part 552 is moved in the direction of spacing away from the plunger 510, and the locking of the plunger 510 by the lock part 552 is canceled.
   In this instance, the spring 520 in the compressed state exerts its elastic force to move the plunger 510 in the direction of the tip end. The movement of the plunger 510 in the direction of the tip end causes the puncture needle 5 to move in the direction of the tip end, and the needle tip 511 of the needle body 51 passes through the tip end opening 31 of the casing 3 to protrude from the tip end of the chip 1, thereby puncturing the skin (surface) of the fingertip.
   Besides, in this instance, in the chip 1, the hub 52 of the puncture needle 5 is moved while being supported on the casing 3 at two positions (the fitting part 531 and the projected part 541). Therefore, unintentional shifts of the puncture needle 5 relative to the casing 3 are efficiently rectified, and the puncture needle 5 is moved in the direction of the tip end with high rectilinearity. By this, an increase in the pain in the patient attendant on unintentional shifting of the needle tip 511 of the needle body 51 can be prevented favorably.
   In addition, the puncturing means 500 is provided with a spring (not shown) for pushing back the plunger 510 in the direction of the base end, and the spring pushes back the plunger 510 in the direction of the base end after the puncturing of a fingertip with the puncture needle 5. By the elastic force of the spring 520 and the elastic force of the pushing-back spring, the plunger 510 is made to repeat the movement in the direction of the tip end and the movement in the direction of the base end, and, after a while, the plunger 510 is left still at the position where the elastic force of the spring 520 and the elastic force of the pushing-back spring balance each other. In this instance, the needle tip 511 of the needle body 51 is contained in the chip 1. Thus, the needle tip 511 of the needle body 51 is designed not to protrude from the tip end of the chip 1 at other times than the time of puncturing, so that mistaken damaging of a skin or the like is obviated, and high safety is realized.
[4] Next, the component measuring instrument 100 with the chip 1 mounted therein (mounted thereto) is once placed on a desk or the like, and the periphery of the site of puncture of a fingertip with the puncture needle 5 is massaged with a finger of the other hand or the like, to cause blood to flow out of the puncture site.
[5] Subsequently, the component measuring instrument 100 is again gripped, and the blood point adhesion part 725 of the chip 1 is brought into contact with the blood being protuberant on the puncture site as a result of the operation in [4] above.
   Upon contact with the blood point adhesion part 725, the blood is introduced via the blood inflow port 741 into the first blood transfer channel 744, and is transferred in the first blood transfer channel 744 by capillarity. Then, the blood transferred in the first blood transfer channel 744 fills sufficiently the space at the boundary part between the first blood transfer channel 744 and the second blood transfer channel 745, and is thereafter drawn up toward the blood outflow port 742 due to the capillarity in the second blood transfer channel 745.
   In this instance, since the convex part 743 is provided on the bottom surface of an end portion, on the second blood transfer channel 745 side, of the first blood transfer channel 744 so as to protrude along the axial direction of the second blood transfer channel 745 (toward the blood transfer direction), the space at the boundary part between the first blood transfer channel 744 and the second blood transfer channel 745 is swiftly filled with the blood, and the blood is speedily transferred into the second blood transfer channel 745, to be drawn up toward the blood outflow port 742.
   Then, the blood transferred in the second blood transfer channel 745 is supplied through the blood outflow port 742 to the test paper placing part 721, and is supplied to the test paper 73 while spreading radially in the gap 756.
   When the blood is supplied to the test paper 73, glucose in the blood and the reagent react with each other at the test paper 73, resulting in coloration according to the amount of glucose.
[6] The coloration at the test paper 73 is detected by the measuring means 900. The measuring means 900 irradiates the test paper 73 with light emitted from the light emitting device 920, then the reflected light is received by the light receiving device 930, and is subjected to photo-electric conversion. Then, an analog signal according to the amount of light received is outputted from the light receiving device 930, the signal is amplified as desired, and is converted into a digital signal by the A/D converter, and the digital signal is inputted to the control means 1100.
[7] The control means 1100 performs a predetermined arithmetic processing based on the digital signal, besides, performs corrections such as temperature correction calculation and hematocrit value correction calculation, as required, and determines the glucose level (blood sugar level) in the blood, i.e., determines the blood sugar level quantitatively. Next, the blood sugar level thus determined is displayed on the display unit 1200, whereby the blood sugar level can be grasped.
   According to this method, the blood in an amount necessary and sufficient for measurement can be sampled speedily and securely, and the blood sugar level (the amount of a predetermined component in the blood) can be accurately measured while using a smaller amount of blood.
[8] Next, with the ejecting mechanism 800 operated, the eject pin 810 is moved in the direction of the tip end, to push the flange 39 in the direction of the tip end. By this, the chip 1 is moved relative to the component measuring instrument 100 in the direction of the tip end, and is detached from the chip mounting part 310 (the component measuring instrument 100).
   In this case, when the casing 3 is moved in the direction of the tip end, the puncture needle 5 connected to the puncturing means 500 is relatively moved toward the tip end side, and the fitting part 531 is fitted into the fitting part 35. Besides, substantially simultaneously with this or before or after this, the connection between the connection part 542 of the puncture needle 5 and the holder part 530 of the puncturing means 500 is canceled.
   Thus, in the chip 1 detached from the component measuring instrument 100, the fitting part 35 and the fitting part 531 are fitted to each other either substantially wholly or partially, and the puncture needle 5 is fixed relative to the casing 3.
[9] Next, the lid body 8 is mounted to a tip end portion of the chip 1, as required, and the chip 1 is thrown away.

While the humor sampling implement and the method of humor sampling according to the present invention have been described above based on the embodiments shown in the figures, the invention is not limited to the embodiments.

The configurations of the parts of the humor sampling implement according to the present invention can be replaced by arbitrary configurations inasmuch as they can display the same functions as those of the original.

For example, while a puncture needle integrated type humor sampling implement in which a puncture needle and a detection unit are integrated has been described as one example in the above embodiments, the humor sampling implement of the invention may be one not provided with the puncture needle and the peripheral members, i.e., one composed of only the above-mentioned detection unit.

In addition, while the second humor transfer channel has been one constituting the cross-sectional area gradually decreasing part in the above embodiments, the cross-sectional area gradually decreasing part may be provided in the first humor transfer channel, and may be provided in each of the first and second humor transfer channels. In other words, the cross-sectional area gradually decreasing part can be provided at an arbitrary position in the humor transfer channel. Besides, the whole part of the humor transfer channel may be composed of the cross-sectional area gradually decreasing part.

In addition, for example, while the configuration in which the puncture needle is supported by the casing at two positions in the longitudinal direction thereof at the time of moving the puncture needle relative to the casing has been adopted in the above embodiments, the puncture needle in the present invention may be supported by the casing at three or more positions. This ensures that the rectilinearity of movement of the puncture needle is more enhanced.

Besides, while a system in which the humor transfer channel is composed of two transfer channels has been described in the above embodiments, the humor transfer channel may be composed of one transfer channel or be composed of three or more transfer channels.

In addition, the humor transfer channel is not limited to the bent one but may be one which is curved in the course of the humor transfer direction.

Besides, blood has been described as a representative of the humor to be sampled in the above embodiments, the humor to be sampled in the present invention is not limited to blood but may be, for example, urine, sweat, lymph, cerebrospinal fluid, bile, saliva or the like.

In addition, glucose (blood sugar level) has been described as a representative of the component to be measured in the above embodiments, the component to be measured in the present invention is not limited to glucose (blood sugar level) but may be, for example, various sugars, cholesterol, lactic acid, hemoglobin (occult blood), uric acid, creatinine, various proteins, inorganic ions such as sodium ion, or the like.

Besides, the measuring means has been described as one for measuring the amount of a predetermined component in the above embodiments, the measuring means in the present invention may be one for measuring the property of a predetermined component, and may be one for measuring the amount and property of a predetermined component.

In addition, a detection unit in which coloration occurs through a reaction of a predetermined component of humor with a reagent, i.e., a detection unit which is applicable to the system of optically detecting a predetermined component (coloration system), has been described in the above embodiments, a detection unit applicable to an electrode type method (a system of electrically detecting a predetermined component) may also be adopted in the present invention. In that case, the detection unit is provided with electrodes, and an appropriate combination of at least one redox enzyme of the above-mentioned enzymes and at least one of electron acceptors such as potassium ferricyanide, ferrocene derivatives, quinone derivatives, and metallic complexes is used as a reagent for reaction with a predetermined component.

### Industrial Applicability

According to the present invention, the collected humor can be transferred to the detection unit more securely and speedily. Therefore, the component measurement can be efficiently conducted, without the trouble that the humor stagnates in the humor transfer channel with the result that the humor sampling implement must be discarded wastefully or that the patient is subjected again to humor sampling. In addition, by the simple structure of providing a convex part and without using other special equipment, the above-mentioned effect can be favorably displayed, so that a reduction in manufacturing cost can be contrived, and applicability to expendable use mode can be obtained. Therefore, the present invention has industrial applicability.

## Claims

1. A humor sampling implement comprising:
a main frame part having a humor transfer channel provided to collect humor through a humor inflow port and transfer said humor to a humor outflow port; and
a detection part provided at said main frame part to detect a predetermined component of said humor transferred through said humor transfer channel; wherein
said main frame part is provided with a convex part arranged so as to be in overlapped relationship with said detection part in plan view and protruding in said humor transfer channel toward said humor outflow port.

2. The humor sampling implement as set forth in claim 1, wherein said convex part is provided at a position corresponding substantially to a center of said detection part.

3. The humor sampling implement as set forth in claim 1 or 2, wherein
said humor transfer channel comprises a first humor transfer channel opening to said humor inflow port, and a second humor transfer channel connected to said first humor transfer channel, said second humor transfer channel being different from said first humor transfer channel in a direction humor transfer; and
said convex part is provided at an end portion on humor outflow port side of said first humor transfer channel of said main frame part so as to protrude in said second humor transfer channel.

4. The humor sampling implement as set forth in claim 3, wherein the direction of humor transfer in said first humor transfer channel and the direction of humor transfer in said second humor transfer channel are substantially orthogonal to each other.

5. The humor sampling implement as set forth in claim 3, wherein V₁/V₂ is in a range of from 0.04 to 0.7, where V₁ [mm³] is a volume of said convex part, and V₂ [mm³] is an inside volume of said second humor transfer channel.

6. The humor sampling implement as set forth in claim 1, wherein said main frame part has a lower member, and an upper member which is positioned on said lower member and which, together with said lower member, defines a part of said humor transfer channel.

7. The humor sampling implement as set forth in claim 1, wherein
said main frame part has a lower member, and an upper member which is positioned on said lower member and which, together with said lower member, defines a part of said humor transfer channel having a first humor transfer channel opening to said humor inflow port and a second humor transfer channel connected to said first humor transfer channel, a direction of humor transfer in said second humor transfer channel being substantially orthogonal to that in said first humor transfer channel;
said convex part is provided at an end portion on a humor outflow port side of said first humor transfer channel of said main frame part so as to protrude in said second humor transfer channel and is provided at a position corresponding substantially to a center of said detection unit; and
V₁/V₂ is in a range of from 0.04 to 0.7, where V₁ [mm³] is a volume of said convex part, and V₂ [mm³] is an inside volume of said second humor transfer channel.

8. A method of humor sampling, wherein a humor sampling implement as set forth in claim 1 is used.

9. A method of humor sampling, comprising the step of collecting humor through a humor inflow port of a humor sampling implement, said humor sampling implement comprising:
a main frame part having a humor transfer channel provided to collect humor through said humor inflow port and transfer said humor to a humor outflow port; and
a detection unit provided in said main frame part to detect a predetermined component of said humor transferred through said humor transfer channel; wherein
said main frame part is provided with a convex part arranged so as to be in overlapped relationship with said detection unit in plan view and protruding in said humor transfer channel toward said humor outflow port.
